# EUROPEAN PATENT APPLICATION

(11) **EP 3 885 427 A1**
(43) Date of publication of application: **29.09.2021**
(21) Application number: 19888142.7
(22) Date of filing: 25.01.2019
(51) Int. Cl.: C12M 1/00, C12M 1/42, C12M 1/34, A61M 1/36

(54) **APPARATUS FOR DELIVERING TARGET MATERIAL USING EXTRACORPOREAL SHOCK-WAVES**

(30) Priority: 22.11.2018 KR 20180145023
(71) Applicant: Exollence Biotechnology, Seoul 07985 (KR)
(72) Inventor: KWON, Ki Hwan, Seoul 06009 (KR)
(74) Representative: Dr. Gassner & Partner mbB
(86) International application number: PCT/KR2019/001058
(87) International publication number: WO 2020/105800

(57) **Abstract**

An apparatus for delivering a target substance according to the present invention comprises: a tube through which a solution including cells or extracellular vesicles and a target substance passes; and a shock-wave generator which is arranged on one side of the tube and applies extracorporeal shock-waves to the solution, thereby inserting the target substance into the cells or the extracellular vesicles. In such an apparatus for delivering a target substance, by introducing a target substance into cells or extracellular vesicles in a tube by applying extracorporeal shock-waves, delivering a target substance into a large amount of cells or extracellular vesicles is rapidly performed, and thus it is industrially easy to mass-produce a therapeutic agent.

## Description

### [Technical Field]

The present disclosure relates to a target substance delivering device, and more specifically, to a target substance delivering device capable of effectively delivering a target substance into cells or extracellular vesicles using an extracorporeal shock-wave.

### [Background Art]

A shock wave refers to a single continuous sound wave generated by a specific sound wave generator, and has an amplitude of a high peak pressure up to 100 MPa, and has a short duration of less than 1 µs, and may be delivered to a specific target site at an energy density in a range of 0.005 to 0.32 mJ/mm².

High energy extracorporeal shock-wave lithotripsy (ESWL) refers to a treatment scheme in which a pressure of 35 to 100 MPa is applied to a specific part of a human body. Since the high energy ESWL has been used to break down stones in kidneys and bile ducts, the high energy ESWL has been used as a new treatment scheme in various fields (Chaussy, C. et al. First clinical experience with extracorporeally induced destruction of kidney stones by shock waves. J Urol 127, 417.420 (1982)). Recently, the extracorporeal shock-wave lithotripsy has been used to treat musculoskeletal diseases. Further, it has been reported that ESWL is effective in anti-inflammatory action and blood flow increase.

Extracellular vesicles are nano vesicles having a size of several tens to hundreds of nm and made of a double lipid membrane, and are composed of substances having biological activity such as proteins, lipids, and genes. In the past, such extracellular vesicles were considered to be debris secreted from cells. However, recently, as clinical significance of extracellular vesicles emerges, various studies thereon have been conducted. In particular, exosomes which are spherical vesicles discharged from cells have various information such as proteins and DNA of a parent cell. Thus, cancer diagnosis markers and sensors using the exosomes as biomarkers are being actively developed. In an example, there is a research result that suggests possibility of diagnosis of glioblastoma based on detection of a gene of a modified form of EGFRVIII in extracellular vesicles present in a patient's blood. In addition, there is a research result that identifies presence of prostate cancer biomarkers PCA-3 and TMPRSS2:ERG via genomic analysis of cancer cell-derived extracellular vesicles among extracellular vesicles in an urine of patients. In addition, a method for diagnosing an inflammatory disease by analyzing an expression level of an inflammatory disease-related gene present in an extracellular vesicles in a body fluid is disclosed in Korean Patent No. 10-1704828.

Further, in Korean Patent No. 10-1719569 prior to the present application, the present applicant set forth a method for producing extracellular vesicles containing a target substance, based on identification that production and secretion of extracellular vesicle such as exosomes, ectosomes, micro vesicles, or apoptotic bodies into which a desired target substance is introduced increased in cells treated with extracorporeal shock-wave. However, the above patent document does not disclose a device capable of producing a therapeutic agent in large quantities by efficiently delivering a target substance into cells or extracellular vesicle.

### [Disclosure]

### [Technical Purpose]

The present disclosure has been proposed to solve all the problems of the prior art. A purpose of the present disclosure is to provide a target substance delivering device capable of mass-producing therapeutic agents by effectively delivering a target substance into cells or extracellular vesicles using extracorporeal shock-waves.

### [Technical Solution]

A first aspect of the present disclosure provides a target substance delivering device including: a tube through which a solution containing cells or extracellular vesicles; and a target substance passes; and a shock-wave generator disposed on one side face of the tube to apply an extracorporeal shock-wave to the solution such that the target substance is inserted into the cells or the extracellular vesicles.

In this connection, the target substance delivering device according to the present disclosure refers to a device for inserting a target substance into a cell or extracellular vesicle. The cell may include all of various differentiated cells or undifferentiated cells known in the art as long as the cell may contain and deliver the target substance using the extracorporeal shock-wave treatment. For example, the differentiated or undifferentiated cell may include all of a body cell, a cancer cell, an organ tissue cell, an induced pluripotent stem cell, or an embryonic stem cell.

Further, the target substance delivering device according to the present disclosure is preferably a device for inserting a target substance into various extracellular vesicles. The extracellular vesicles include at least one selected from the group consisting of exosomes, ectosomes, micro vesicles, apoptotic bodies, vesicle-type substances derived from cells or living organisms, and vesicle-type substances artificially constructed using extracellular vesicles or cells.

Further, the target substance includes at least one selected from the group consisting of nucleic acids, proteins and compounds.

Further, the device further includes: a controller connected to the shock-wave generator, and configured to control an energy level and a period of the extracorporeal shock-wave to be applied to the solution, in which the controller is connected to the tube and configured to control a flow rate of the solution in the tube.

Further, the tube includes a plurality of tubes arranged in at least one of series and parallel manners and connected to each other.

A second aspect of the present disclosure provides a target substance delivering device including: a tube through which a solution containing cells or extracellular vesicles passes; a nozzle connected to one side of the tube to inject a target substance into the solution; and a shock-wave generator disposed on one side face of the tube to apply an extracorporeal shock-wave to the solution such that the target substance is inserted into the cells or the extracellular vesicles.

A third aspect of the present disclosure provides a target substance delivering device including: a tube through which a solution containing cells or extracellular vesicles passes; a shock-wave generator disposed on one side face of the tube to apply an extracorporeal shock-wave to the solution; a chamber connected to one end of the tube, in which the chamber accommodates therein the solution to which the extracorporeal shock-wave has been applied; and a nozzle connected to one side of the chamber to inject a target substance into the solution accommodated in the chamber, in which the target substance is inserted into the cells or the extracellular vesicles contained in the solution to which the extracorporeal shock-wave has been applied.

A fourth aspect of the present disclosure provides a target substance delivering device including: a separator configured to separate blood discharged from a blood vessel of a patient into cells and a plasma liquid containing extracellular vesicles excluding the cells; a connector connected to the separator, in which the cells separated by the separator pass through the connector; a tube connected to the separator, in which the plasma liquid separated by the separator passes through the tube; a nozzle connected to at least one of the connector and the tube, in which the nozzle is configured to inject a target substance into at least one of the plasma liquid and the cells separated by the separator; a shock-wave generator disposed on one side face of at least one of the connector and the tube, in which the shock-wave generator is configured to apply an extracorporeal shock-wave to at least one of the cells and the plasma liquid such that the target substance is injected into at least one of the cells and the extracellular vesicle; and a circulator connected to the connector and the tube, in which the circulator is configured to circulate back at least one of the cells into which the target substance is inserted and the plasma liquid containing the extracellular vesicles into which the target substance is inserted into the blood vessel of the patient.

Further, the device further includes: a controller connected to the shock-wave generator, and configured to control an energy level and a period of the extracorporeal shock-wave to be applied from the shock-wave generator to at least one of the cells and the plasma liquid, in which the controller is connected to at least one of the connector and the tube and configured to control a flow rate of at least one of the cells and the plasma liquid.

Further, the tube includes a plurality of tubes arranged in at least one of series and parallel manners and connected to each other.

### [Advantageous Effects]

The target substance delivering device according to the present disclosure may apply the extracorporeal shock-wave to the tube to introduce the target substance into the cells or extracellular vesicles, so that the process of delivering the target substance to a large amount of cells or extracellular vesicles is made quickly, thereby achieving mass-production of therapeutic agents commercially.

Further, the target substance delivering device according to the present disclosure may adjust the flow rate of the solution, and the energy level and the period of the extracorporeal shock-wave based on the type of the solution, and the types and concentrations of the cells or extracellular vesicle, and of the target substance. Thus, using a single device, various target substances may be delivered to various cells or extracellular vesicles as efficiently as possible.

Further, the target substance delivering device according to the present disclosure may separate the patient's blood in real time into the cells and the plasma liquid containing extracellular vesicles, then insert the target substance into at least one of the cells and the extracellular vesicles contained in the plasma liquid, and then may directly inject back at least one of the cells into which the target substance is inserted; and the plasma liquid containing the extracellular vesicles into which the target substance is inserted into the patient's blood vessel. In this way, the target substance may be delivered to patient's target cells or target organ without side effects due to an immune response.

### [Description of Drawings]

FIG. 1 is a schematic diagram showing a target substance delivering device according to a first embodiment of the present disclosure.
FIG. 2 is a schematic diagram showing a target substance delivering device according to a second embodiment of the present disclosure.
FIG. 3 is a schematic diagram showing a target substance delivering device according to a third embodiment of the present disclosure.
FIG. 4 is a schematic diagram showing a target substance delivering device according to a fourth embodiment of the present disclosure.

### [Modes of the Invention]

Hereinafter, embodiments of the present disclosure will be described in detail with reference to the accompanying drawings. It should be noted that in assigning reference numerals to elements of each drawing, the same elements have the same numerals, even though they are shown on different drawings. Further, when it is determined that detailed descriptions thereof obscure the gist of the present disclosure, the detailed description thereof will be omitted. Further, embodiments of the present disclosure will be described below, but a technical idea of the present disclosure is not limited thereto and may be implemented by a person skilled in the art.

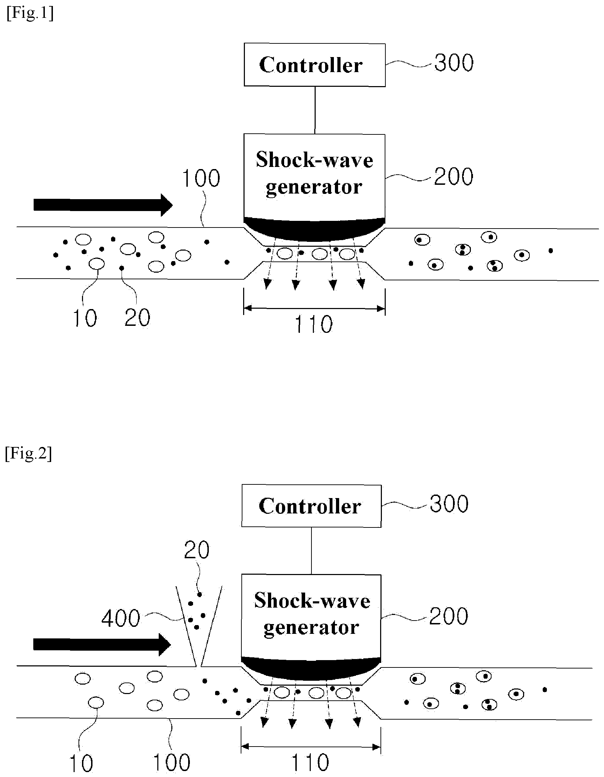

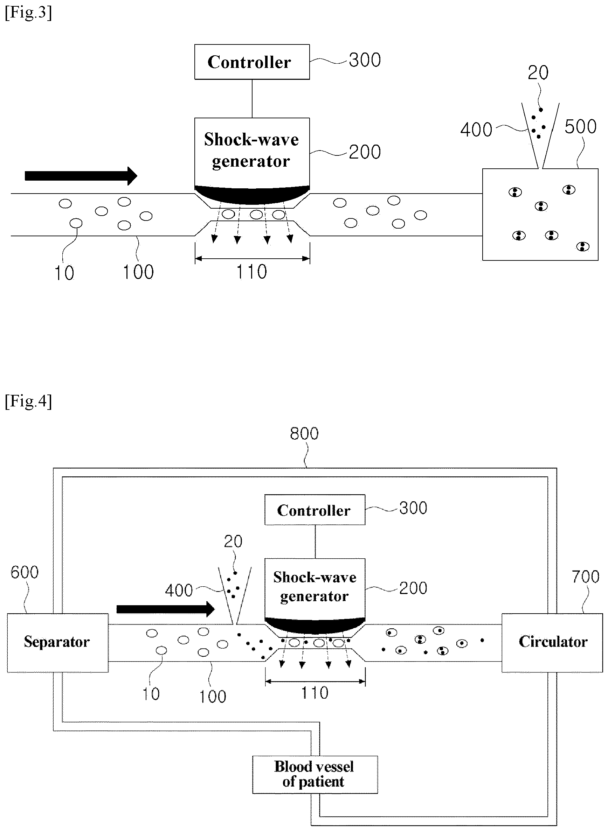

FIG. 1 is a schematic diagram showing a target substance delivering device according to a first embodiment of the present disclosure. FIG. 2 is a schematic diagram showing a target substance delivering device according to a second embodiment of the present disclosure. FIG. 3 is a schematic diagram showing a target substance delivering device according to a third embodiment of the present disclosure. FIG. 4 is a schematic diagram showing a target substance delivering device according to a fourth embodiment of the present disclosure.

Hereinafter, a target substance delivering device according to the first embodiment of the present disclosure will be described with reference to FIG. 1.

A tube 100 is hollow such that a solution containing cells or extracellular vesicles 10; and a target substance 20 passes therethrough. In this connection, a cross section of the tube 100 may be formed into a circle, an oval, a square, a hexagon, a triangle, or the like. The tube 100 may be made of silicon or a metal such as stainless steel, or a plastic such as polystyrene, but is not limited thereto.

In one example, the solution passing through the inside of the tube 100 may be a buffer suitable for containing the cells or extracellular vesicles 10 and the target substance 20. For example, the solution may be saline, but is not limited thereto.

In one example, among the cells or the extracellular vesicles 10 contained in the solution, the cells may include all of various differentiated cells or undifferentiated cells known in the art as long as the cells may contain and deliver the target substance under extracorporeal shock-wave treatment. For example, the differentiated or undifferentiated cells may include all of a body cell, a cancer cell, an organ tissue cell, an induced pluripotent stem cell, or an embryonic stem cell.

Further, among the cells or extracellular vesicles 10 contained in the solution, the extracellular vesicles may be derived from any organism or cell. The organism may be a plant or an animal. Further, the extracellular vesicles may be cells separated from a subject. That is, the extracellular vesicles may be derived from any animal including humans and non-human mammals, or may be derived from various types of immune cells and tumor cells.

Specifically, the extracellular vesicles may be at least one selected from the group consisting of exosomes, ectosomes, micro vesicles and apoptotic bodies, vesicle-type substances derived from cells or living organisms, and vesicle-type substances that have been artificially fabricated using extracellular vesicles or cells.

In one example, the target substance 20 contained in the solution may be at least one substance selected from the group consisting of nucleic acids, proteins and compounds. In this connection, the nucleic acid may include any nucleic acid that exists in nature or may be prepared artificially. Preferably, the nucleic acid may include DNA, RNA, microRNA (miRNA), small RNA (smRNA), small interfering RNA (siRNA), piwi-interacting RNA (piRNA), small nucleolar RNA (snoRNA), t-RNA-derived small RNA (tsRNA), small rDNA-derived RNA (srRNA), small nuclear RNA (U-RNA) and long noncoding RNA (IncRNA). The nucleic acid may include a plasmid vector containing a specific sequence. However, the present disclosure is not limited thereto.

A shock-wave generator 200 is placed on one side of the tube 100, and is configured to apply the extracorporeal shock-wave to the solution passing through tube 100 to insert the target substance 20 into the cells or extracellular vesicles 10. Thus, the target substance 20 may be inserted into the cells or extracellular vesicles 10 to which the extracorporeal shock-wave has been applied.

In this connection, an energy level and an application period of the extracorporeal shock-wave applied from the shock-wave generator 200 to the tube 100 may vary based on a distance between the shock-wave generator 200 and the tube 100, types and concentrations of the cells or extracellular vesicle, and a type and a concentration of the target substance. For example, the extracorporeal shock-wave may be applied in an energy range of 0.05 to 0.9 mJ/mm², specifically, 0.05 to 0.89 mJ/mm², and more specifically, 0.05 to 0.70 mJ/mm², but is not limited thereto.

A controller 300 may be connected to the shock-wave generator 200 and may adjust the energy level and the period of the extracorporeal shock-wave to be applied from the shock-wave generator 200 to the solution passing through the tube 100. That is, the controller 300 calculates an optimal energy level and an optimal application period that may vary according to the above-described various conditions and transmits the calculated result to the shock-wave generator 200. The shock-wave generator 200 may be configured to apply the extracorporeal shock-wave based on the optimized energy level and period transmitted from the controller 300 to the solution passing through the tube 100.

Further, the controller 300 may be connected to tube 100 and configured to control a flow rate at which the solution flows in the tube 100. That is, the controller 300 may control the flow rate of the solution, such that the energy level and the period of the extracorporeal shock-wave may be adjusted based on the flow rate. Thus, even when the flow rate of the solution varies depending on the type of solution, and the types and concentrations of the cells or extracellular vesicle, and the target substance, the extracorporeal shock-wave may be applied thereto at the most appropriate energy level and period that correspond to the flow rate. Therefore, it is possible to insert the target substance into the cells or extracellular vesicles at maximum efficiency. As a result, a process of delivering the target substance to a large amount of the cells or extracellular vesicles may be performed quickly, such that it is easy to commercially mass-produce therapeutic agents.

In one example, it is desirable that the tube 100 to which the extracorporeal shock-wave is applied from the shock-wave generator 200 is sealed. In other words, if the tube 100 is not completely sealed, air is introduced into the tube 100 and thus the extracorporeal shock-wave is not completely delivered into the solution. Thus, the process of inserting the target substance into the cells using the extracorporeal shock-wave is not performed properly. Therefore, it is desirable that the tube 100 is completely sealed to prevent air from entering the tube.

Further, a cross-sectional area of the tube 100 may be determined based on the types of the cells or extracellular vesicles and the target substance contained in the solution passing through the tube, and a placement position of the shock-wave generator 200. That is, it is preferable that the cross-sectional area of the tube 100 is sized within a distance that allows the extracorporeal shock-wave generated from the shock-wave generator 200 to be uniformly delivered to the solution passing through the inside of the tube 100.

The extracorporeal shock-wave energy that may reach the inside of the tube 100 decreases as a portion of the shock-wave generator 200 where the extracorporeal shock-wave is generated is further away from the tube 100 by a certain distance. Therefore, this fact may be considered to properly determine the placement position of the shock-wave generator 200 and the cross-sectional area of the tube 100.

The tube 100 may have a narrow portion 110 having a relatively reduced cross-sectional area than those of other portions thereof. Specifically, the narrow portion 110 may be formed only in a portion of the tube 100 where the shock-wave generator 200 is disposed on the tube 100 such that the extracorporeal shock-wave is applied to the portion. That is, the tube 100 may have the narrow portion 110 whose cross-sectional area is reduced as much as possible so that the extracorporeal shock-wave may be applied evenly from the shock-wave generator 200 to the tube.

Further, although not shown, the tube 100 may be formed to have a uniform cross-sectional area along an entire length direction thereof without the narrow portion 110. It is preferable that the overall cross-sectional area of the tube 100 that does not have the narrow portion 110 is sized within a range sized such that the extracorporeal shock-wave is applied as uniformly as possible.

In one example, a method for inducing the flow of the solution in the tube 100 includes a method of using a pressure difference using a pump (not shown), but is not limited thereto.

Further, a flow direction through which the solution passes in the tube 100 shown in FIG. 1 is shown as right direction, but is not limited thereto. The solution may flow in a left direction.

In one example, although not shown, a plurality of tubes 100 may be arranged in at least one of series and parallel manners and may be connected to each other. In this connection, different solutions respectively containing different cells or extracellular vesicles 10 may pass through the different tubes 100, respectively. In this connection, different target substances 20 may be inserted into the different cells or extracellular vesicles 10, respectively.

When the plurality of tubes 100 are arranged in a parallel manner and connected to each other, the process of inserting the target substances 20 into the cells or extracellular vesicles 10 using the extracorporeal shock-wave may be performed simultaneously on the plurality of tubes 100 as connected in parallel to each other, thereby increasing productivity of the therapeutic agent.

Further, when the plurality of tubes 100 are arranged in a series manner and connected to each other, the process in which the target substances 20 are inserted into the cells or extracellular vesicles 10 using the extracorporeal shock-wave may be sequentially performed on the plurality of tubes 100.

Therefore, when continuously injecting the same target substance 20 through a nozzle 400 which will be described later and connected to each of the plurality of tubes 100 as arranged in a series manner and connected to each other, the target substance 20 inserted into the cells or extracellular vesicles 10 may have a higher concentration. Further, when continuously injecting different target substances 20 through different nozzles 400 connected to different tubes 100, respectively, various target substances 20 may be inserted into the cells or extracellular vesicles 10.

As described above, when the plurality of tubes 100 are arranged in at least one of the series and parallel manners and are connected to each other, each of the plurality of tubes 100 may be connected to the controller 300. That is, the controller 300 may be configured to be connected to each of the plurality of tubes 100 to individually adjust the flow rate of each solution passing through each tube 100.

Hereinafter, with reference to FIG. 2, a structure of the target substance delivering device according to the second embodiment of the present disclosure will be described. For convenience of description, descriptions of the same components as in the first embodiment shown in FIG. 1 are omitted. Hereinafter, differences therebetween will be mainly described.

Referring to FIG. 2, the target substance delivering device further includes the nozzle 400. The nozzle 400 may be connected to one side of the tube 100 and may be configured to inject the target substance 20 into the solution passing through the tube 100.

That is, unlike in the first embodiment of FIG. 1 in which the target substance 20 passes through the tube 100 while being contained in the solution containing the cells or extracellular vesicles 10, in the second embodiment of FIG. 2, when the solution containing the cells or extracellular vesicles 10 passes through tube 100, the target substance 20 may be injected into the solution through the nozzle 400.

In this connection, although not shown in detail, the nozzle 400 may be connected to the tube 100 to inject the target substance 20 just before or before the solution containing the cells or extracellular vesicles 10 passes by the shock-wave generator 200. Alternatively, the nozzle 400 may be connected to the tube 100 to inject the target substance 20 while the solution containing the cells or extracellular vesicles 10 passes through a portion of the tube to where the extracorporeal shock-wave is applied. Alternatively, the nozzle 400 may be connected to the tube 100 to inject the target substance 20 immediately after or after the solution containing the cells or extracellular vesicles 10 passes by the shock-wave generator 200.

Further, an amount of the target substance 20 injected into the tube 100 from the nozzle 400 may be adjusted based on the types of the cells or extracellular vesicles 10 and the target substance 20, and the flow rate of the solution. In this connection, the nozzle 400 may have an adjuster (not shown) connected to one side thereof. This adjuster (not shown) may be configured to adjust the amount of the target substance injected through the nozzle 400.

In this way, the target substance delivering device according to the present disclosure may adjust the amount of the target substance 20 injected into the solution passing through the tube 100 through the nozzle 400 connected to the tube 100 and the adjuster (not shown) connected thereto. Therefore, there is an advantage that it is possible to efficiently produce the therapeutic agent while reducing the amount of use of the target substance 20 such as expensive nucleic acids and proteins as much as possible.

In one example, a shape of the nozzle 400 shown in FIG. 2 is illustrative and is not limited thereto. The shape of the nozzle 400 may be modified by any person skilled in the art.

Hereinafter, with reference to FIG. 3, a structure of the target substance delivering device according to the third embodiment of the present disclosure will be described. For convenience of description, descriptions of the same components as in the second embodiment shown in FIG. 2 are omitted. Hereinafter, differences therebetween will be mainly described.

Referring to FIG. 3, the target substance delivering device further includes a chamber 500. The chamber 500 is connected to one end of the tube 100 and accommodates therein the solution to which the extracorporeal shock-wave has been applied.

In this connection, unlike the second embodiment of FIG. 2 in which the nozzle 400 is connected to one side of the tube 100, in the third embodiment of FIG. 3, the nozzle 400 is connected to one side of the chamber 500, and inject the target substance 20 into the received solution in the chamber 500.

That is, when the solution containing the cells or extracellular vesicles 10 passes through the tube 100 while the extracorporeal shock-wave is applied thereto from the shock-wave generator 200, and subsequently, the solution is accommodated in the chamber 500, the target substance 20 is injected into the solution accommodated in the chamber 500 through the nozzle 400. As described above, the target substance 20 injected through the nozzle 400 may be inserted into the cells or extracellular vesicles 10 to which the extracorporeal shock-wave has been applied.

Hereinafter, with reference to FIG. 4, a structure of the target substance delivering device according to the fourth embodiment of the present disclosure will be described. For convenience of description, descriptions of the same components as in the second embodiment shown in FIG. 2 are omitted. Hereinafter, differences therebetween will be mainly described.

Referring to FIG. 4, the target substance delivering device further includes a separator 600, a circulator 700, and a connector 800. The separator 600 may use a plasmapheresis technique in which blood is separated into a cell fraction and a plasma fraction.

The separator 600 may be configured to separate blood discharged from a blood vessel of the patient into cells (not shown); and a plasma liquid containing extracellular vesicles excluding the cells. The plasma liquid separated by the separator 600 passes through the tube 100 connected to the separator 600, while the cells separated by the separator 600 pass through the connector 800 connected to the separator 600.

Although not shown in detail, the nozzle 400 is connected to at least one of the connector 800 and the tube 100 to inject the target substance 20 into at least one of the cells separated by the separator 600 and the plasma liquid separated by the separator 600.

In this connection, although not shown in detail, the shock-wave generator 200 may be disposed on one side of at least one of the connector 800 and the tube 100 to apply the extracorporeal shock-wave to at least one of the cells separated by the separator 600 and the plasma liquid separated by the separator 600.

When the extracorporeal shock-wave is applied thereto, the target substance 20 may be inserted into at least one of the cells passing through the connector 800; and the extracellular vesicles 10 contained in the plasma liquid passing through the tube 100.

The circulator 700 may be connected to at least one of the connector 800 and the tube 100, and may circulate back at least one of the cells into which the target substance 20 is inserted; and the plasma liquid containing the extracellular vesicles 10 into which the target substance 20 is inserted into the blood vessel of the patient.

In this way, the target substance delivering device according to the fourth embodiment of the present disclosure may separate the patient's blood in real time into the cells and the plasma liquid containing extracellular vesicles, then insert the target substance 20 into at least one of the cells and the extracellular vesicles contained in the plasma liquid, and then may directly inject back at least one of the cells into which the target substance 20 is inserted; and the plasma liquid containing the extracellular vesicles 10 into which the target substance 20 is inserted into the patient's blood vessel. In this way, the target substance 20 may be delivered to patient's target cells or target organ without side effects due to an immune response.

As described above, the preferred embodiments of the present disclosure have been described in detail, but the technical scope of the present disclosure is not limited to the above-described embodiments and should be interpreted by the claims. In this connection, those who have acquired ordinary knowledge in this technical field should consider that many modifications and variations are possible without departing from the scope of the present disclosure.

## Claims

1. A target substance delivering device comprising:
a tube through which a solution containing cells or extracellular vesicles; and a target substance passes; and
a shock-wave generator disposed on one side face of the tube to apply an extracorporeal shock-wave to the solution such that the target substance is inserted into the cells or the extracellular vesicles.

2. A target substance delivering device comprising:
a tube through which a solution containing cells or extracellular vesicles passes;
a nozzle connected to one side of the tube to inject a target substance into the solution; and
a shock-wave generator disposed on one side face of the tube to apply an extracorporeal shock-wave to the solution such that the target substance is inserted into the cells or the extracellular vesicles.

3. A target substance delivering device comprising:
a tube through which a solution containing cells or extracellular vesicles passes;
a shock-wave generator disposed on one side face of the tube to apply an extracorporeal shock-wave to the solution;
a chamber connected to one end of the tube, wherein the chamber accommodates therein the solution to which the extracorporeal shock-wave has been applied; and
a nozzle connected to one side of the chamber to inject a target substance into the solution accommodated in the chamber.
wherein the target substance is inserted into the cells or the extracellular vesicles contained in the solution to which the extracorporeal shock-wave has been applied.

4. The device of one of claims 1 to 3, wherein the device further comprises:
a controller connected to the shock-wave generator, and configured to control an energy level and a period of the extracorporeal shock-wave to be applied to the solution,
wherein the controller is connected to the tube and configured to control a flow rate of the solution in the tube.

5. The device of one of claims 1 to 3, wherein the tube includes a plurality of tubes arranged in at least one of series and parallel manners and connected to each other.

6. A target substance delivering device comprising:
a separator configured to separate blood discharged from a blood vessel of a patient into cells and a plasma liquid containing extracellular vesicles;
a connector connected to the separator, wherein the cells separated by the separator pass through the connector;
a tube connected to the separator, wherein the plasma liquid separated by the separator passes through the tube;
a nozzle connected to at least one of the connector and the tube, wherein the nozzle is configured to inject a target substance into at least one of the plasma liquid and the cells separated by the separator;
a shock-wave generator disposed on one side face of at least one of the connector and the tube, wherein the shock-wave generator is configured to apply an extracorporeal shock-wave to at least one of the cells and the plasma liquid such that the target substance is injected into at least one of the cells and the extracellular vesicle; and
a circulator connected to the connector and the tube, wherein the circulator is configured to circulate back at least one of the cells into which the target substance is inserted and the plasma liquid containing the extracellular vesicles into which the target substance is inserted into the blood vessel of the patient.

7. The device of claim 6, wherein the device further comprises:
a controller connected to the shock-wave generator, and configured to control an energy level and a period of the extracorporeal shock-wave to be applied from the shock-wave generator to at least one of the cells and the plasma liquid,
wherein the controller is connected to at least one of the connector and the tube and configured to control a flow rate of at least one of the cells and the plasma liquid.

8. The device of claim 6, wherein the tube includes a plurality of tubes arranged in at least one of series and parallel manners and connected to each other.

9. The device of one of claims 1 to 3 and claim 6, wherein the extracellular vesicles include at least one selected from a group consisting of exosomes, ectosomes, microvesicles, apoptotic bodies, vesicle-type substances derived from cells or living organisms, and vesicle-type substances artificially constructed using extracellular vesicles or cells.

10. The device of one of claims 1 to 3 and claim 6, wherein the target substance includes at least one selected from a group consisting of nucleic acids, proteins and chemical compounds.
